# EUROPEAN PATENT SPECIFICATION

(11) **EP 2 604 681 B1**
(45) Date of publication and mention of the grant of the patent: **08.02.2017**
(21) Application number: 11009797.9
(22) Date of filing: 12.12.2011
(51) Int. Cl.: C12M 3/00, C12M 3/06, G01N 33/483, G01N 33/50, G01N 33/487

(54) **Use of an arrangement for impedance measurements across the surface of a tubular membrane manufactured from a semipermeable material**
Verwendung einer Vorrichtung zur Impedanzmessung auf der Oberfläche einer rohrförmigen, aus halbdurchlässigem Material hergestellten Membran
Utilisation d'un agencement de mesures d'impédance sur la surface d'une membrane tubulaire fabriquée en matériau semi-perméable

(43) Date of publication of application: 19.06.2013
(73) Proprietor: nanoAnalytics GmbH, 48149 Münster (DE)
(72) Inventor: Anczykowski, Boris, Dr., 48143 Münster (DE); Anczykowski, Bernhard, 48157 Münster (DE)
(74) Representative: Herzog, Fiesser & Partner Patentanwälte PartG mbB

(56) References cited:
- EP-A1- 2 302 353
- JP-A- 2008 191 084
- WEGENER J ET AL: "Automated multi-well device to measure transepithelial electrical resistances under physiological conditions", BIOTECHNIQUES, INFORMA HEALTHCARE, US, vol. 37, no. 4, 1 January 2004 (2004-01-01), pages 590-597, XP008126784, ISSN: 0736-6205
- NICHOLAS FERRELL ET AL: "A microfluidic bioreactor with integrated transepithelial electrical resistance (TEER) measurement electrodes for evaluation of renal epithelial cells", BIOTECHNOLOGY AND BIOENGINEERING, vol. 107, no. 4, 15 June 2010 (2010-06-15) , pages 707-716, XP55026683, ISSN: 0006-3592, DOI: 10.1002/bit.22835
- PAUL A. VOGEL ET AL: "Microfluidic Transendothelial Electrical Resistance Measurement Device that Enables Blood Flow and Postgrowth Experiments", ANALYTICAL CHEMISTRY, vol. 83, no. 11, 1 June 2011 (2011-06-01), pages 4296-4301, XP55021633, ISSN: 0003-2700, DOI: 10.1021/ac2004746

## Description

The invention relates to the use of an arrangement for impedance measurements across the surface of a tubular membrane manufactured from a semi-permeable material and to a process for measuring the impedance across the surface of a tubular membrane manufactured from a semi-permeable material.

Cell culture systems are a useful tool for studying the behavior of cells *in vitro.* However, in an ordinary static cell culture system (i.e. a cell culture system in which cells are cultured on an optionally permeable substrate and in which the cells are covered with a cell culture medium which is periodically exchanged with fresh medium) the "microenvironment" in which the cells are cultured often does not completely represent the microenvironment the cells are exposed to *in vivo.* Endothelial cells, for example, which in the form of a thin layer line the interior surface of blood vessels, form an interface between circulating blood in the lumen and the rest of the vessel wall. *In vivo,* the blood flow continuously passes over the cell surface and the shear forces created thereby influence the differentiation and thus the physiological properties of the cell layer. For *in vitro* studies in which these effects cannot be neglected, experimental setups other than the above described static cell culture systems are required.
Moreover, cell culture systems are also used to grow cells to densities and/or structures approaching those of living tissues. Such approaches have included the use of suspension cultures, as well as the use of small pieces of cellulose sponge and various circumfusion techniques. However, it has been found that there are certain basic problems which must be overcome in order to grow an organ-like structure *in vitro.* One of problems the person skilled in the art in confronted with is that components of the medium must diffuse through the cell layers to reach all cells, and this diffusion, of course, becomes more difficult as the thickness of the cell layer increases.

In order to overcome the above mentioned restrictions of static cell culture systems, so called "dynamic cell culture systems" have been described in which, for example, cells are grown in reactors comprising tubular capillaries manufactured from a semi-permeable membrane. Through the tubular capillaries as well as through the voids of the reactor a cell culture medium can continuously be passed through, thereby allowing oxygen and nutrients to be supplied to the cells while metabolic waste products are continuously eliminated. Such a cell culture approach is, for example, disclosed in US 3,821,087 and US 3,883,393 or is commercially available under the trade name "CellMax^{®} Bioreactor" from Spectrum^{®} Laboratories Inc., California, USA. A further dynamic system which has been developed for culturing brain capillary endothelial cells on the surface of tubular capillaries manufactured from a semi-permeable membrane is the DIV-BBB system from Flowcel Inc., Ohio, USA. In this dynamic model nutrients are continuously pumped through the models luminal space. This adds shear stress on the endothelial cells in the model, and allows these cells to have a higher degree of differentiation and stronger tight junctions between each endothelial cell. Thus, the DIV-BBB exemplifies an *in vitro* model that more accurately describes the *in vivo* conditions of the BBB (blood brain barrier).

In special cell culture systems the properties of the cells are analyzed by electric measurements. For this purpose the cells are grown on the surface of small and planar gold-film electrodes, which are deposited on the bottom of a cell culture dish. The AC impedance of the cell-covered electrode is then measured at one or several frequencies as a function of time. Due to the insulating properties of their membranes the cells behave like dielectric particles so that the impedance increases with increasing coverage of the electrode until a confluent (i.e. continuous) layer of cells is established. In confluent cell layers the measured impedance is related to the three-dimensional structure and shape of the cells. If cell morphology changes or, in the case of endothelial or epithelial cells, changes in the structure of the tight junctions occur, the current pathways through and around the cell bodies change as well, leading to a corresponding increase or decrease of impedance. Thus, by recording time-resolved impedance measurements, changes in the morphology of the cells or in the structure of tight junctions can be followed in real time and in a non-invasive manner. This technique can be employed for bioanalytic purposes. In contrast to other approaches for studying the morphology of cells, the impedance analysis does not require the use of markers such as fluorescence labeled antibodies.

A device for analyzing the impedance across a layer of cells cultured on a permeable membrane is disclosed in DE 10 2009 052 673 A1. However, experimental setups as disclosed in this prior art document are tailored for analyzing the impedance in a static cell culture system. They cannot be used for analyzing cells that are cultivated in dynamic cell culture systems such as the CellMax^{®} Bioreactor or the DIV-BBB system. Nevertheless, attempts to analyze the impedance across a layer of cells in dynamic systems have also been disclosed in the prior art. In the DIV-BBB system, for example, the transendothelial electrical resistance (TEER) can be measured via electrodes that are provided within the hollow fiber cartridge system.

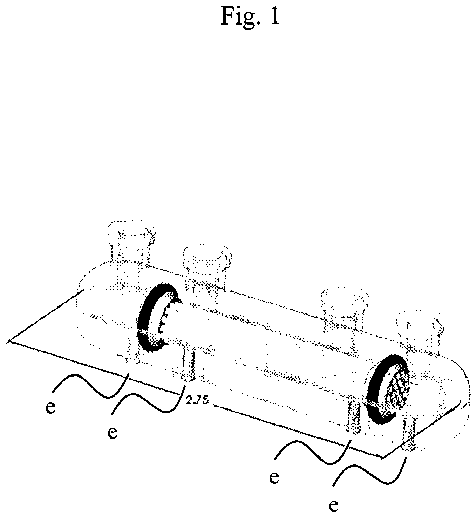

The disadvantage of the DIV-BBB system, however, lies in the fact that due the special punctual arrangement of the electrodes (see figure 1) an inhomogeneous electrical field is created along the longitudinal axis of the hollow fibers by means of which a precise determination of the TEER or the capacity is not possible.

In general, the object of the invention consists in contributing to overcoming the disadvantages arising from the prior art in the context of the impedance analysis in cell culture systems, especially in dynamic cell culture systems.

A further object consists in the use of an arrangement that can be used for impedance analysis of a dynamic cell culture system such as the CellMax^{®} Bioreactor. The arrangement should enable the person skilled in the art to measure the impedance across a cell layer while the cell layer is exposed to a shear stress in a dynamic cell culture system. Contrary to the DIV-BBB system, the arrangement should allow the precise determination of electrical parameters like the TEER or the capacity across the surface of a hollow fiber membrane or across a layer of cells cultured on the surface of a hollow fiber membrane over a significant portion of the total length of the hollow fibers, preferably over the total length of the hollow fiber.

An arrangement for impedance measurement across the surface of a tubular membrane is described in JP 2008/191084. This arrangement is used for desalting of water by means of electrodialysis.

A contribution to the solution of these objects is made by an arrangement for impedance measurements across the surface of a tubular membrane manufactured from a semi-permeable material, as defined in claim 1.

The arrangement comprises as a first component a) a tubular outer electrode having a longitudinal axis. Usually, the tubular outer electrode has an inner diameter in the range from 1 mm to 100 mm, preferably in the range from 3 mm to 20 mm. The wall thickness of the tubular outer electrode is preferably within the range from 100 µm to 2.5 mm, preferably within the range from 200 µm to 1 mm. Thus, the outer diameter of the tubular electrode is usually in the range from 1.2 mm to 105 mm, preferably in the range from 3.4 mm to 22 mm.

As the outer electrode has to be electrically conductive, it is either completely manufactured from an electrically conductive material or it is, at least at its inner tubular surface (i.e. the surface that is facing towards the inner electrode in the arrangement according to the present invention), coated with an electrically conductive material. Suitable electrically conductive materials comprise metals such as steal, silver, gold or platinum, wherein steal, especially stainless steel, is particularly preferred, or alloys. Furthermore, the outer electrode can also be manufactured from, or at least the inner surface of the outer electrode can be coated with, an electrically conductive polymer. Examples of conductive polymers include *cis*-polyacetylene, trans-polyacetylene, poly(para-phenylene), polythiophenes, polypyrrole, poly(*para*-phenylen-vinylene) or poly-aniline.

In a particular preferred embodiment of the arrangement the tubular outer electrode is manufactured from stainless steel and has an inner diameter in the range from 0.5 mm to 10 mm, preferably 1 mm to 5 mm and a wall thickness in the range from 100 µm to 1 mm, preferably in the range from 250 µm to 500 µm.

The outer electrode can also comprise a viewing window through which the at least one tubular membrane, especially the surfaces thereof, can be studied by, for example, light microscopy. The surface area of such a viewing window can be in the range from 0.25 cm² to 10 cm², preferably from 1 cm² to 5 cm². Such a viewing window may be manufactured of glass or a transparent plastic such as acrylic glass.

The arrangement comprises as a second component b) at least one tubular membrane manufactured from a semi-permeable membrane and having a longitudinal axis, the at least one tubular membrane being located within the at least one tubular outer electrode in such a way that the longitudinal axis of the at least one tubular membrane is substantially parallel to the longitudinal axis of the tubular outer electrode. Usually, the at least one tubular membrane has an inner diameter in the range from 10 µm to 49 mm, preferably in the range from 500 µm to 10 mm. The semi-permeable membrane from which the at least one tubular membrane is manufactured preferably has a wall thickness in the range from 10 µm to 5 mm, particularly preferred from 100 µm to 1 mm.

Suitable tubular membranes are, for example, the so called *"hollow fiber membranes"* (HFM) which are typically used in micro or ultrafiltration devices used to purify water. These tubular membranes can, for example, be obtained from the Membrana GmbH, Wuppertal, Germany. Particularly suitable hollow fiber membranes are, for example, the UltraPES™ or the MicroPES™ polyethersufone (PES) hollow-fiber membranes or the AccurelPP™ polypropylene (PP) hollow fiber membrane that are available from the Membrana GmbH.

Usually, the semi-permeable membrane from which the at least one tubular membrane is manufactured is based on a material selected from the group consisting of a thermoplastic polymer, preferably polypropylene, polyethylene or polysulfone, cellulose or a cellulose derivative, preferably cellulose acetate and regenerated cellulose, sulfonated polyethersulfone polymers and fluorinated polymers, preferably polyvinylidene fluoride (PVDF) or polytetrafluoroethylene (PTFE, Teflon).

Preferably, the semi-permeable membrane from which the at least one tubular membrane is manufactured is a porous semi-permeable membrane having a pore size in the range from 1 nm to 10 µm, preferably from 0.2 µm to 3 µm.

According to a particular embodiment of the arrangement
- the semi-permeable membrane from which the at least one tubular membrane is manufactured has a wall thickness in the range from 10 µm to 5 mm, preferably from 100 µm to 1 mm, and
- the semi-permeable membrane from which the at least one tubular membrane is a porous semi-permeable membrane having a pore size in the range from 1 nm to 10 µm, preferably from 0.2 µm to 3 µm.

The arrangement comprises as a third component c) at least one inner electrode in the form of a wire, tube or rod having a longitudinal axis, the at least one inner electrode being located within the at least one tubular membrane in such a way that the longitudinal axis of the at least one inner electrode is substantially parallel to the longitudinal axis of the at least one tubular membrane. Usually, the at least one inner electrode has an outer diameter in the range from 0.1 mm to 45 mm, preferably in the range from 0.2 mm to 2 mm.

As the at least one inner electrode also has to be electrically conductive, it is preferably manufactured from one of those electrically conductive materials that have already been mentioned in connection with the tubular outer electrode. Preferably, the at least one inner electrode is manufactured from a metal such as steal, silver, gold or platinum, wherein steal, especially stainless steel, is particularly preferred, or alloys.

Preferably, the tubular outer electrode, the at least one tubular membrane and the at least one inner electrode have a length in the range from 0.5 cm to 200 cm, preferably in the range from 2 cm to 20 cm, wherein the length of the tubular outer electrode, the at least one tubular membrane and the at least one inner electrode within a given arrangement can be identical or different from one another.

According to a particular embodiment of the arrangement
- the tubular outer electrode, the at least one tubular membrane and the at least one inner electrode have a length in the range from 0.5 cm to 200 cm, preferably from 2 cm to 20 cm,
- the tubular outer electrode has an inner diameter in the range from 1 mm to 100 mm, preferably in the range from 3 mm to 20 mm,
- the at least one tubular membrane has an inner diameter in the range from 10 µm to 49 mm, preferably in the range from 0.5 mm to 10 mm, and
- the at least one inner electrode has an outer diameter in the range from 0.1 mm to 45 mm, preferably in the range from 0.2 mm to 2 mm.

According to a further embodiment of the arrangement according to the present invention, it is furthermore preferred that the tubular outer electrode, the at least one tubular membrane and the at least one inner electrode are dimensioned and arranged in such a way that the at least one tubular membrane is sandwiched between the tubular outer electrode and the at least one inner electrode over at least 10 %, preferably at least 25 %, more preferably at least 50 %, more preferably at least 75 % and most preferably at least 95 % of the total length of the at least one tubular membrane. The expression "the at least one tubular membrane is sandwiched between the tubular outer electrode and the at least one inner electrode over a certain part of the total length of the at least one tubular" is meant to describe an arrangement wherein in this part the outer surface of the at least one tubular membrane is covered by the inner surface of the outer electrode and the inner surface of the at least one tubular membrane is covered by the outer surface of the at least one inner electrode.

The arrangement may, besides the tubular outer electrode, the at least one tubular membrane and the at least one inner electrode, comprise further parts, such as, for example, fittings that are located at the proximal end and the distal end of the arrangement. These fittings correspond to the fittings 13a shown in Figure 2 of US 3,821,087, the disclosure of which is incorporated herein by reference. These fittings are preferably connectable to a tube and form the closure of the proximal and distal end of the tubular outer electrode. By connecting such fittings with a tube it is possible to guide a liquid, for example a nutrient medium or an aqueous salt solution, through the arrangement. If the at least one tubular membrane is arranged in such a way that its distal and proximal ends are located close to these fittings, it is also possible to guide the liquid, or at least a predominant part thereof, through the inner tubular space of the at least one tubular membrane manufactured from a semi-permeable membrane. Preferred fittings are those comprising a Luer-Lock fitting, for example a Male-Luer-Lock or a Female-Luer-Lock.

The arrangement may further comprise an inlet port located near the proximal end of the arrangement and an outlet port located near the distal end of the arrangement, the inlet port and the outlet port being configured to allow the entry (inlet port) and the discharge (outlet port) of a liquid medium through the casing of the tubular outer electrode. These ports correspond to the ports 15 shown in Figure 2 of US 3,821,087. By connecting the inlet port and the outlet port with a tube it is possible to guide a further liquid stream, for example a nutrient medium or an aqueous salt solution, through the inner tubular space of the outer electrode and thus across the outer surface of the at least one tubular membrane manufactured from a semi-permeable membrane. The inlet port and the outlet port can also comprise a Luer-Lock fitting, for example a Male-Luer-Lock or a Female-Luer-Lock.

According to a particular embodiment the arrangement comprises a plurality of tubular capillaries manufactured from a semi-permeable membrane being located within the outer electrode, wherein in at least one of the tubular capillaries, preferably in all of the tubular capillaries, an inner electrode is located. The arrangement may also comprise fixation means for fixing the at least one tubular membrane within the inner space of the outer electrode in such a way that the tubular capillaries are located substantially parallel to the outer and the at least one inner electrode. These fixation means correspond to end pieces 14 in figure 2 of US 3,821,087.

According to a further particular embodiment the arrangement further comprises at least one protective sheathing in the form of a tubular membrane manufactured from a semi-permeable membrane, the at least one protective sheathing covering the surface of the at least one inner electrode and having a longitudinal axis, the at least one inner electrode being located within the at least one protective sheathing and the at least one protective sheathing being located within the tubular membrane and the axis of the at least one inner electrode, the at least one tubular membrane and the at least one protective sheathing the being substantially parallel to each other. By means of such a protective sheathing the inner electrode can be protected. If, for example, a cell suspension is introduced into the arrangement for the purpose of culturing cells on the surface of the semi-permeable membrane, it can be avoided that the cells directly attach to the inner electrode. Preferably, the inner diameter of the tubular membrane serving as the protective sheathing is slightly larger than the diameter of the inner electrode. The wall thickness of the tubular membrane serving as the protective sheathing as well as the material out of which this protective tubular membrane is manufactured preferably correspond to the wall thickness and the materials that have already been mentioned in connection with the tubular capillaries serving as the second component b) of the arrangement.

According to a further particular embodiment the arrangement further may comprise, optionally in addition to the above described at least one protective sheathing of the at least one inner electrode, a further protective sheathing in the form of tubular membrane manufactured from a semi-permeable membrane, this further protective sheathing covering the inner surface of the tubular outer electrode (i.e. the surface that is facing towards the at least one inner electrode) and having a longitudinal axis, the at least one tubular membrane being located within this further protective sheathing and the further protective sheathing being located within the tubular outer electrode and the axis of the tubular outer electrode, the at least one tubular membrane and the further protective sheathing the being substantially parallel to each other. By means of such a further protective sheathing the inner surface of the tubular outer electrode can also be protected. If, for example, a cell suspension is introduced into the arrangement for the purpose of culturing cells on the surface of the semi-permeable membrane, it can be avoided that the cells directly attach to the tubular outer electrode. Preferably, the outer diameter of the tubular membrane serving as the other protective sheathing is slightly smaller than the inner diameter of the tubular outer electrode.

Moreover, the arrangement can comprise contacts by means of which the outer electrode and the at least one inner electrode can be connected with an electronic device such as an Impedance Analyzer.

A contribution to the solution of the objects to be solved by the present invention is also made by a process for measuring the impedance across the surface of a tubular membrane manufactured from a semi-permeable material, the process comprising the process steps as defined in claim 12.

If in the process according to the present invention cells should be cultured on one of the surface sides or on both surface sides of the semi-permeable material of which the tubular membrane is manufactured, it is possible to separately culture the cells onto these surfaces by placing the tubular membrane in an appropriate culture flask containing a suspension of the cells and to place the tubular membrane into the arrangement after the cells have been grown onto the surface of the semi-permeable membrane to the desired density. It is, however, also possible (and preferred) to culture the cells on the surface of the semi-permeable membrane out of which the tubular membrane is manufactured while the tubular membrane is located in the arrangement. In this case, the inner tubular space of the outer electrode, the inner tubular space of the tubular membrane or the inner tubular space of the outer electrode and the inner tubular space of the tubular membrane (depending on the side or sides of the tubular membrane onto which the cells should adhere) are filled with a cell suspension for a time sufficient to enable the adhesion onto the surface of the semi-permeable membrane. Once the cells are attached to the surface of the semi-permeable membrane, the cell suspension can be substituted by an appropriate nutrient medium.

The arrangement is particularly suitable for co-culturing two or more different cell types. If, for example, cells are cultured on both surface sides of the semi-permeable material of which the tubular membrane is manufactured, it is possible to culture one cell type on one side and another cell type on the other side of the semi-permeable membrane. As an example of such a co-culture system a co-culture of astrocytes and brain capillary endothelial cells can be mentioned.
In the second step of the process according to the present invention, the void within the tubular outer electrode, the void within the at least one tubular membrane and the pores of the semi-permeable material are filled with an electrically conductive liquid, such as, for example, a nutrient medium, a salt solution or a cell suspension comprising a salt solution or a nutrient medium in which cells are suspended.

Preferably, the electrically conductive liquid is guided through the void within the tubular outer electrode and the void within the at least one tubular membrane. The pores of the semi-permeable material out of which the at least one tubular membrane is manufactured can be filled with the electrically conductive liquid simply by means of capillary forces or contacting the semi-permeable material with the electrically conductive liquid under a certain pressure. As the semi-permeable material us usually manufactured from a hydrophobic material, it is also possible to first contact the semi-permeable material with, for example, ethanol and to subsequently substitute the ethanol in the pores of the semi-permeable material with the electrically conductive liquid.
The electrically conductive liquids flow through the outer electrode and/or the at least one tubular membrane while the impedance is measured. Depending on the particular arrangement of the components of the arrangement different approaches of guiding electrically conductive liquids through the void within the tubular outer electrode and the void within the at least one tubular membrane can be realized.
According to a first embodiment of the process according to the present invention, the electrically conductive liquid only flows through the outer electrode (i.e. the void within the outer electrode that is not occupied by the at least one tubular membrane) while the impedance is measured, and the void within the tubular membrane is filled with an electrically conductive liquid that is not streaming.

According to a second embodiment of the process according to the present invention, the electrically conductive liquid only flows through the tubular membrane while the impedance is measured, and the outer electrode (i.e. the void within the outer electrode that is not occupied by the at least one tubular membrane) is filled with an electrically conductive liquid that is not streaming.

According to a third embodiment of the process according to the present invention the electrically conductive liquid flows through the outer electrode (i.e. the void within the outer electrode that is not occupied by the at least one tubular membrane) and the at least one tubular membrane in a counter flow direction while the impedance is measured.

According to a fourth embodiment of the process according to the present invention the electrically conductive liquid flows through the outer electrode (i.e. the void within the outer electrode that is not occupied by the at least one tubular membrane) and the at least one tubular membrane in a concurrent flow direction while the impedance is measured.

If, as previously described, the arrangement further comprises a protective sheathing in the form of tubular membrane manufactured from a semi-permeable membrane, the protective sheathing covering the surface of the inner electrode and having a longitudinal axis, even more embodiments of the process according to the present invention can be considered. For example, in addition to the presence of cells on one of the surface sides or on both surface sides of the semi-permeable material of which the tubular membrane is manufactured, cells can also be cultured on the outer side (i.e. the side that is facing towards the inner side of the tubular membrane) of the sheathing. If a further protective sheathing is used to protect the inner surface of the tubular outer electrode, cells can also be cultured on the inner side (i.e. the side that is facing towards the inner electrode) of this further protective sheathing.

In process step c) of the process according to the present invention the impedance is measured across the surface of the at least one tubular membrane. In order to measure the impedance, the tubular outer electrode and the at least on inner electrode are connected to an appropriate impedance analysis device, such as an Impedance Analyzer. For measuring the impedance across the surface of a tube manufactured from a semi-permeable material, an alternating voltage with a small amplitude, the frequency of which is alternated in the course of the measurement, is applied. The impedance of the systems is determined as a function of the frequency. The principle of the impedance spectroscopy is based on the determination of the electric impedance of the membrane systems of the cultured cells as a function of the frequency of the exciter signal. The measured total electrical impedance typically comprises contributions stemming from ohmic resistances and capacities.

The impedance can be measured while the electrically conductive liquids flow through the outer electrode and/or the at least one tubular membrane as described above. It is, however, also possible to measure the impedance in a static system in which the void within the tubular outer electrode and the void within the at least one tubular membrane are filled with the electrically conductive liquid, but in which there is no flow of these liquids in these voids at the point of time at which the impedance is actually measured. Thus, the arrangement can be used for culturing cells, in which medium can be exchanged or drugs or other components such as particles can be delivered to the cells by guiding fresh medium or solutions of drugs and particles through these voids by means of pumps, but wherein thereafter the impedance analysis is measured under static conditions.
By means of the above described impedance measurement across the surface of the at least one tubular membrane different investigations can be performed. If the arrangement is used, for example, as a filtration or dialysis device, the formation of deposits or biofilms on the surface sides of the semi-permeable membrane can be followed and analyzed. If the arrangement is used in a cell culture system, both the progress in the formation of a cell layer as well as the influence of active drugs on the cultured cells can be followed and analyzed. Especially the TEER (transepithelial electrical resistance or transendothelial electrical resistance) and the capacity that can be derived from the impedance analysis can be used to get information about the morphology of the cells and the formation of, for example, tight junctions between adjacent cells.

It may be advantageous to move the surface of the tubular membrane (i.e. the surface that is serving as a substrate for the cell cultures under investigation) relative to the liquid filling the void between the tubular outer electrode and the tubular membrane and between the tubular membrane and the inner electrode. This can, for example, be realized by rotating the at least one tubular membrane around its longitudinal axis or by moving the at least one tubular membrane parallel to the longitudinal axis of the outer electrode, wherein this movement can, for example, be a longitudinal and periodic (back and forth) movement. The resulting shear forces which are exerted on the cells allow to mimic *in vivo* flow conditions.

The following figure serves for exemplary elucidation of the invention and are not to be interpreted as a restriction.
Figure 1 show the arrangement of the electrodes used for analyzing the impedance in the DIV-BB system with electrodes e.
Fig. 2 shows the general arrangement 1 of the basic components (tubular outer electrode 2, tubular membrane 3 and inner electrode 4 of the arrangement 1 in a cross sectional view.
Fig. 3 shows the possibilities A, B and C of culturing cells in the arrangement shown in fig. 2 in a cross sectional view.
Fig. 4 shows the possibilities A, B, C and D of guiding streams of an electrically conductive liquid through the arrangement 1 shown in fig. 2.
Fig. 5 shows a particular embodiment of the arrangement 1 in which the inner electrode 4 is covered by a protective sheathing 7a in a cross sectional view.
Fig. 6 shows the possibilities A, B and C of culturing cells in the arrangement shown in fig. 5 in a cross sectional view.
Fig. 7 shows the possibilities A, B, C and D of guiding streams of an electrically conductive liquid through the arrangement 1 shown in fig. 5.
Fig. 8 shows a further particular embodiment of the arrangement 1 according to the present invention in which the inner surface of the tubular outer electrode 2 is covered by a protective sheathing 7b in a cross sectional view.
Fig. 9 shows the possibilities A, B and C of culturing cells in the arrangement shown in fig. 8 in a cross sectional view.
Fig. 10 shows the possibilities A, B, C and D of guiding streams of an electrically conductive liquid through the arrangement 1 shown in fig. 8.
Fig. 11 shows a further particular embodiment of the arrangement 1 in which the inner electrode 4 as well as the tubular outer electrode 2 are covered by a protective sheathings 7a, 7b and in a cross sectional view.
Fig. 12 shows the possibilities A, B and C of culturing cells in the arrangement shown in fig. 11 in a cross sectional view.
Fig. 13 shows the possibilities A, B, C and D of guiding streams of an electrically conductive liquid through the arrangement 1 shown in fig. 11.
Fig. 14 shows a further particular embodiment of the arrangement 1 according to the present invention similar to the one shown in Figure 11.
Fig. 15 shows, in a cross sectional view, a further particular embodiment of the arrangement 1 in which four tubular capillaries 3 manufactured from a semi-permeable membrane are arranged within the inner space of the tubular outer electrode 2, an inner electrode 4 being provided in each of the four tubular capillaries 3.
Figure 16 shows a further particular embodiment of the arrangement 1 in a view from the top (picture on the top), in a side view (picture in the middle) and in a cross sectional side view (picture on the bottom).
Figure 17 shows a possibility of moving the outer surface of the tubular membrane relative to the inner surface of the tubular outer electrode.

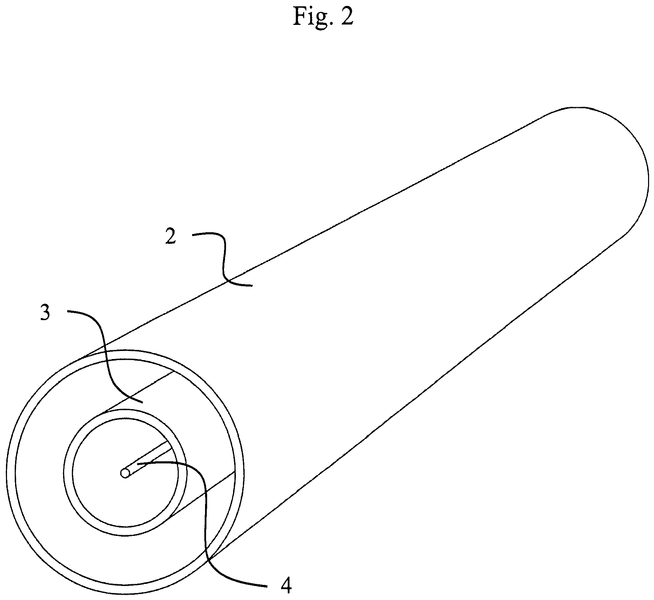

Fig. 2 shows the general arrangement 1 of the basic components. The arrangement comprises a tubular outer electrode 2, at least one tubular membrane 3 and at least one inner electrode 4. As can be seen in figure 1, the at least one inner electrode 4 is located within the at least one tubular membrane 3 in such a way that the longitudinal axis of the at least one inner electrode 4 is substantially parallel to the longitudinal axis of the at least one tubular membrane 3. The at least one tubular membrane 3 is located within the tubular outer electrode 2 in such a way that the longitudinal axis of the tubular membrane 3 is substantially parallel to the longitudinal axis of the tubular outer electrode 2.

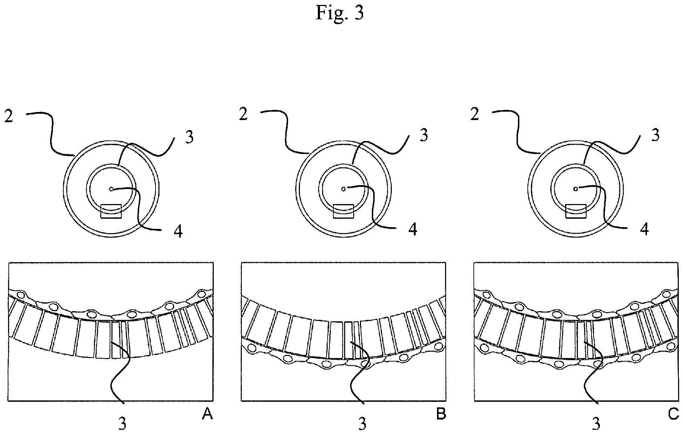

Fig. 3 shows the possibilities of culturing cells on the surface of the semi-permeable membrane out of which the tubular capillaries 3 are manufactured in the arrangement shown in fig. 2 in a cross sectional view. As can be seen in figure 3, the cells can be cultured
- on the surface side of the tubular membrane 3 that is facing towards the inner electrode 4 (see the enlarged detail in figure 3 on the left),
- on the surface side of the tubular membrane 3 that is facing towards the inner side of the outer electrode 2 (see the enlarged detail in figure 3 in the middle), or
- on the surface side of the tubular membrane 3 that is facing towards the inner side of the outer electrode 2 and the surface side that is facing towards the inner side of the outer electrode 2 (see the enlarged detail in figure 3 on the right).

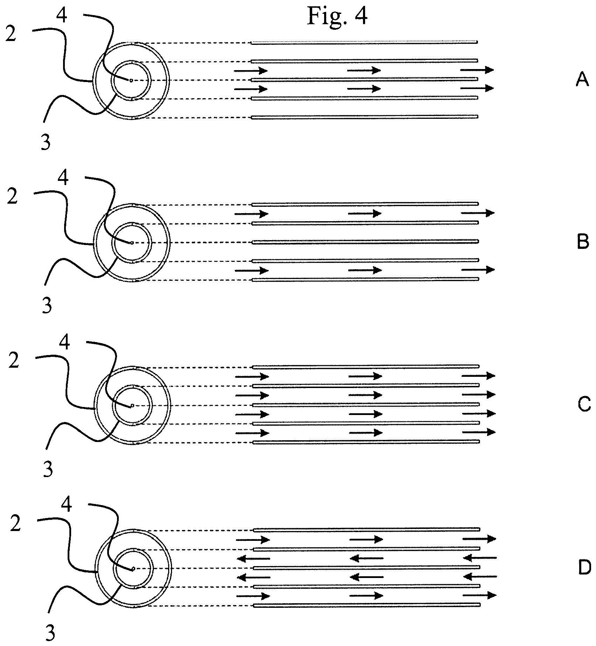

Fig. 4 shows the possibilities of guiding streams of an electrically conductive liquid through the arrangement 1 shown in fig. 2. As can be seen in figure 4,
- the electrically conductive liquid can only flow through the outer electrode 2 (i.e. the void within the outer electrode 2 that is not occupied by the at least one tubular membrane 3) while the impedance is measured, and the void within the tubular membrane 3 is filled with an electrically conductive liquid that is not streaming (see the first illustration in figure 4);
- the electrically conductive liquid flows only flows through the tubular membrane 3 while the impedance is measured, and the outer electrode 2 (i.e. the void within the outer electrode 2 that is not occupied by the at least one tubular membrane 3) is filled with an electrically conductive liquid that is not streaming (see the second illustration in figure 4);
- the electrically conductive liquid flows through the outer electrode 2 (i.e. the void within the outer electrode (2) that is not occupied by the at least one tubular membrane 3) and the at least one tubular membrane 3 in a concurrent flow direction while the impedance is measured (see the third illustration in figure 4);
- the electrically conductive liquid flows through the outer electrode 2 (i.e. the void within the outer electrode 2 that is not occupied by the at least one tubular membrane 3) and the at least one tubular membrane 3 in a counter flow direction while the impedance is measured (see the fourth illustration in figure 4).

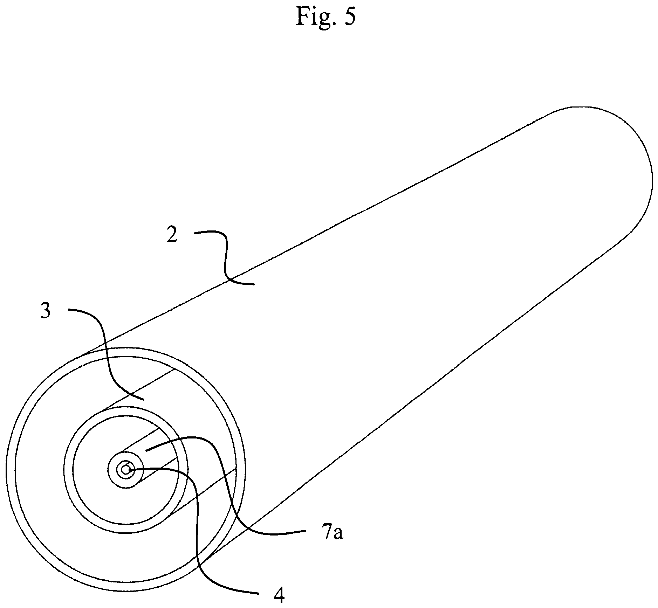

Fig. 5 shows a particular embodiment of the arrangement 1 in which the inner electrode 4 is covered by a protective sheathing 7a in a cross sectional view. As can be seen in figure 5, the protective sheathing 7a is in the form of tubular membrane manufactured from a semi-permeable membrane. The protective sheathing 7a covers the surface of the inner electrode 4. It is, however, not in direct contact with the inner electrode 4. The inner electrode 4 is located within the protective sheathing 7a and the protective sheathing 7a is located within the tubular membrane 3. The axis of the inner electrode 4, the tubular membrane 3 and the protective sheathing 7a are substantially parallel to each other.

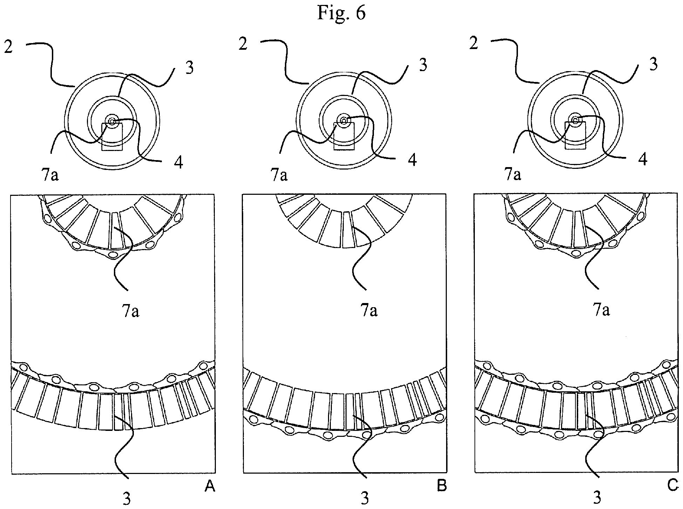

Fig. 6 shows the possibilities of culturing cells on the surface of the semi-permeable membrane out of which the tubular capillaries 3 and the protective sheathing 7a are manufactured in the arrangement shown in fig. 5 in a cross sectional view. As can be seen in figure 6, the cells can not only be present on one of the surface sides or on both surface sides the semi-permeable material out of which the tubular membrane 3 is manufactured, the cells can also be cultured on the outer side (i.e. the side that is facing towards the inner side of the tubular membrane 3) of the sheathing 7a.

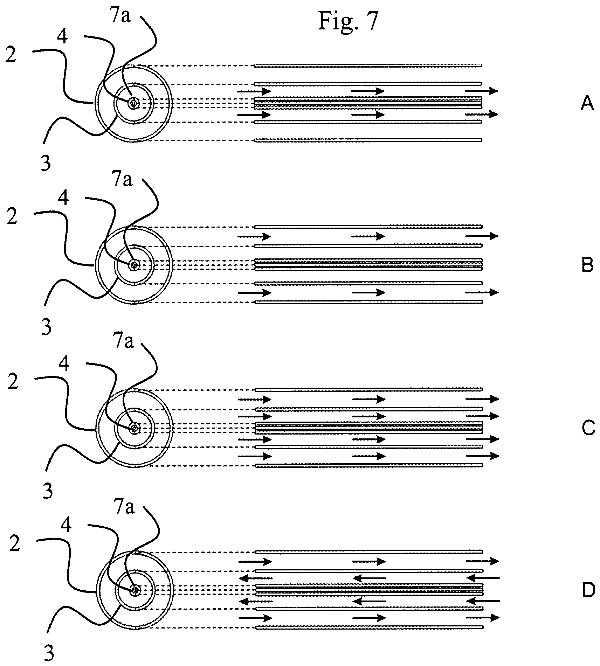

Fig. 7 shows the possibilities of guiding streams of an electrically conductive liquid through the arrangement 1 shown in fig. 5. With respect to these possibilities reference is made to the above description of figure 4.

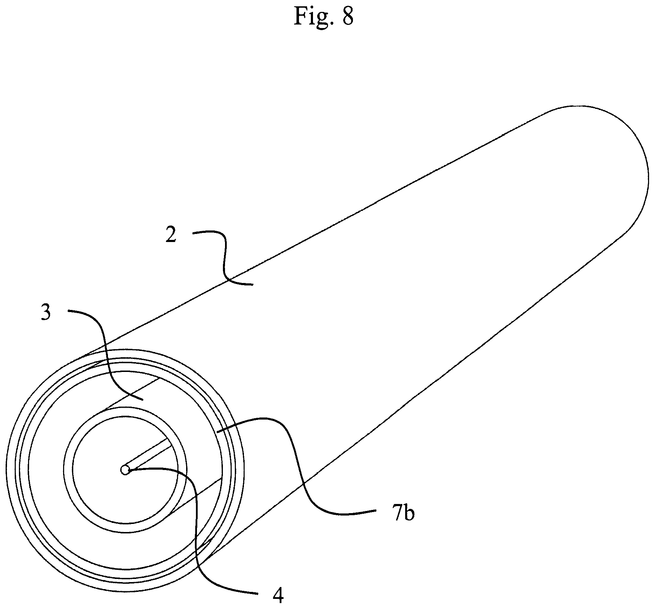

Fig. 8 shows a particular embodiment of the arrangement 1 in which the inner surface of the tubular outer electrode 2 is covered by a protective sheathing 7b in a cross sectional view. As can be seen in figure 8, the protective sheathing 7b is in the form of tubular membrane manufactured from a semi-permeable membrane. The protective sheathing 7b covers the inner surface of the tubular outer electrode 2. It is, however, not in direct contact with the inner surface of the tubular outer electrode 2. The tubular membrane 3 is located within the protective sheathing 7b and the protective sheathing 7b is located within the tubular outer electrode 2. The axis of the tubular outer electrode 2, the tubular membrane 3 and the protective sheathing 7b are substantially parallel to each other.

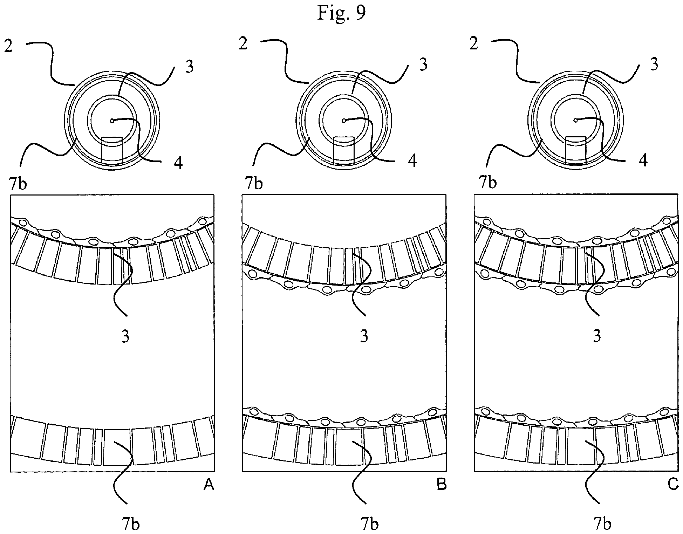

Fig. 9 shows the possibilities of culturing cells on the surface of the semi-permeable membrane out of which the tubular capillaries 3 and the protective sheathing 7b are manufactured in the arrangement shown in fig. 8 in a cross sectional view. As can be seen in figure 9, the cells can not only be present on one of the surface sides or on both surface sides of the semi-permeable material out of which the tubular membrane 3 is manufactured, the cells can also be cultured on the inner side (i.e. the side that is facing towards the tubular membrane 3) of the sheathing 7b.

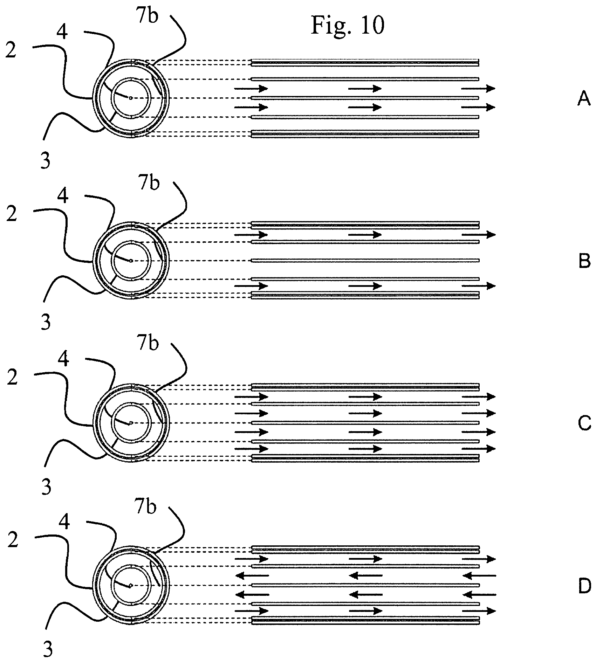

Fig. 10 shows the possibilities of guiding streams of an electrically conductive liquid through the arrangement 1 shown in fig. 8. With respect to these possibilities reference is made to the above description of figure 4.

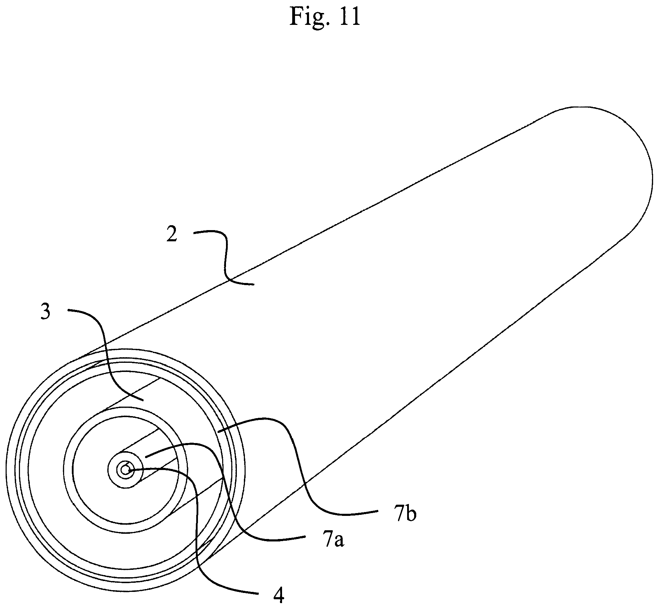

Fig. 11 shows a further particular embodiment of the arrangement 1 in which the inner electrode 4 as well as the tubular outer electrode 2 are covered by a protective sheathings 7a, 7b and in a cross sectional view. The protective sheathing 7a covers the surface of the inner electrode 4 and the protective sheathing 7b covers the inner surface of the tubular outer electrode 2. The protective sheathings 7a, 7b are, however, not in direct contact with the inner surface of the tubular outer electrode 2 and the inner electrode 4, respectively. The inner electrode 4 is located within the protective sheathing 7a and the protective sheathing 7a is located within the tubular membrane 3. The protective sheathing 7b is located within the tubular outer electrode 2 and the inner tubular membrane 3 is located within the protective sheathing 7b. The axis of the inner electrode 4, the tubular membrane 3 and the protective sheathings 7a and 7b are substantially parallel to each other.

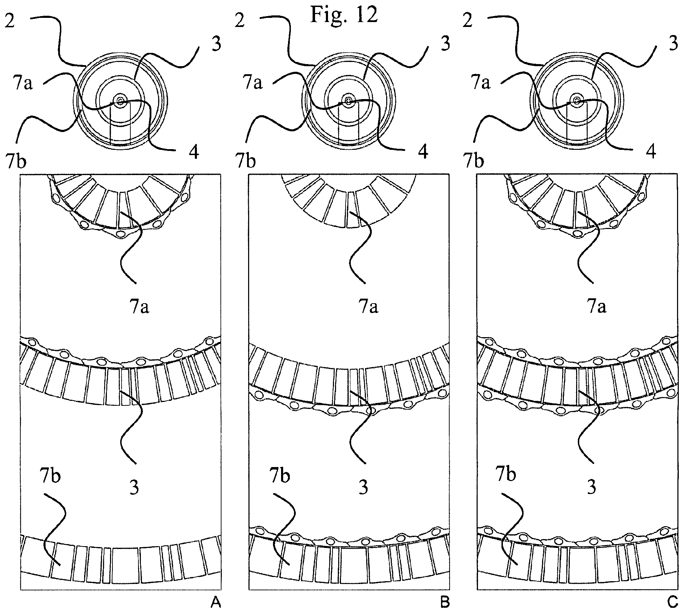

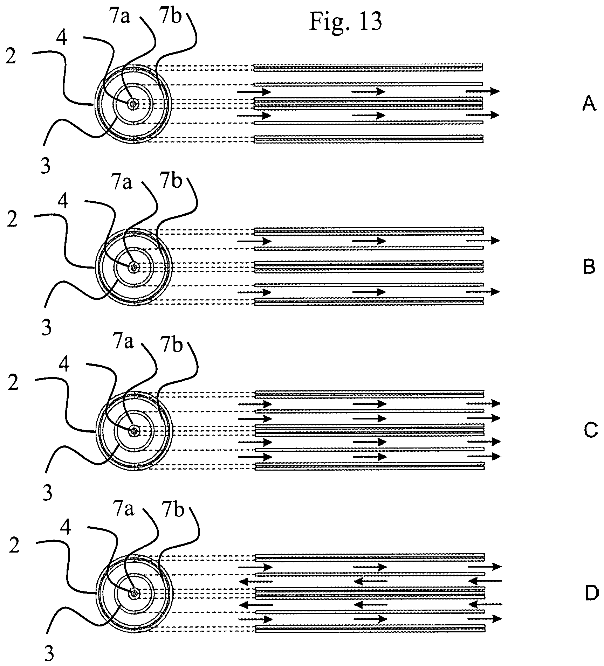

Fig. 12 shows the possibilities A, B and C of culturing cells on the surface of the semi-permeable membrane out of which the tubular capillaries 3 and the protective sheathings 7a and 7b are manufactured in the arrangement shown in fig. 11 in a cross sectional view. Fig. 13 shows the possibilities A, B, C and D of guiding streams of an electrically conductive liquid through the arrangement 1 shown in fig. 11. As can be seen in figure 12, the cells can not only be present on one of the surface sides or on both surface sides of the semi-permeable material out of which the tubular membrane 3 is manufactured, the cells can also be cultured on the outer side (i.e. the side that is facing towards the inner side of the tubular membrane 3) of the sheathing 7a and/or on the inner side (i.e. the side that is facing towards the tubular membrane 3) of the sheathing 7b.

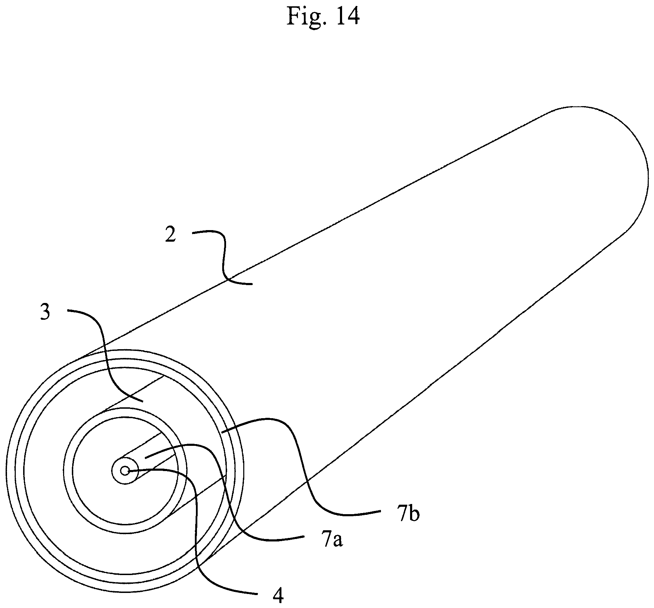

Fig. 14 shows a further particular embodiment of the arrangement 1 similar to the one shown in Figure 11, with the only difference that the protective sheathings 7a, 7b are in direct contact with the surface of the inner electrode 4 and the inner surface of the tubular outer electrode 2, respectively. Such a localization of the protective sheathings in direct contact with the surface or the inner or the outer electrode can, for example, be realized by the process for fabricating fibrous electrochemical cells disclosed in US 5,916,514.

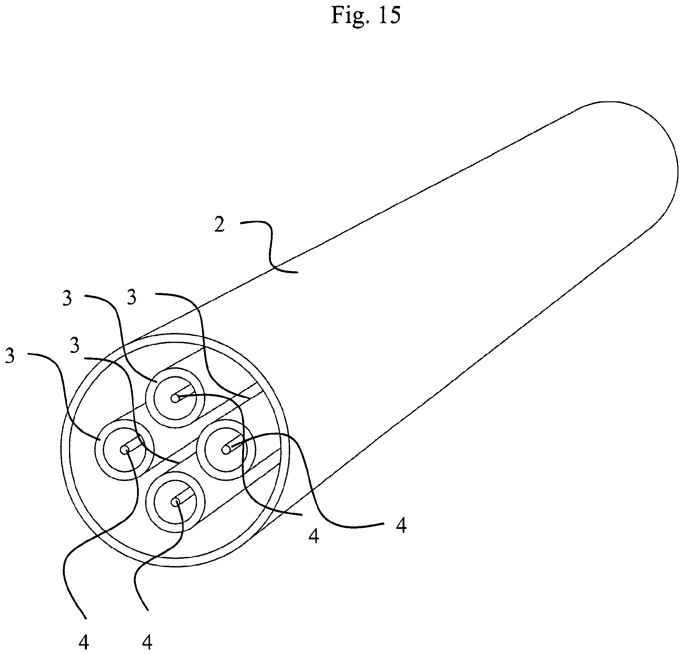

Fig. 15 shows, in a cross sectional view, a further particular embodiment of the arrangement 1 in which four tubular capillaries 3 manufactured from a semi-permeable membrane are arranged within the inner space of the tubular outer electrode 2, an inner electrode 4 being provided in each of the four tubular capillaries 3.

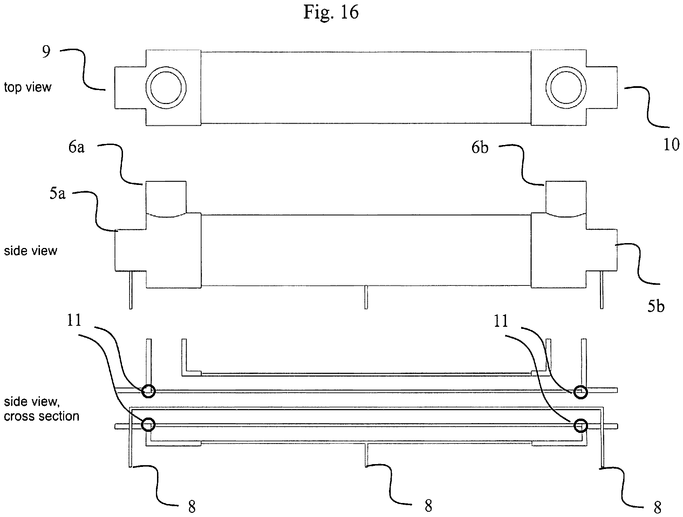

Figures 16 shows a further particular embodiment of the arrangement 1 in a view from the top (picture on the top), in a side view (picture in the middle) and in a cross sectional side view (picture on the bottom), wherein the arrangement 1 further comprises fittings 5a, 5b at the proximal end 9 and the distal end 10 of the arrangement 1, an inlet port 6a located near the proximal end 9 of the arrangement 1 and an outlet port 6b being located near the distal end 10 of the arrangement 1, and contacts 8. The arrangement 1 may further comprise fixation means 11 for fixing the at least one tubular membrane 3 within the inner space of the outer electrode 2 in such a way that the tubular membranes 3 are located substantially parallel to the outer electrode 2 and the at least one inner electrode 4. In case of only one tubular membrane 3, as shown in figure 16, this tubular membrane 3 can, for example, be fixed to the fittings 5a and 5b using appropriated adhesive spots 11. If more than one tubular membrane 3 is located in the arrangement 1, fixation means that correspond to end pieces 14 in figure 2 of US 3,821,087 can be used.

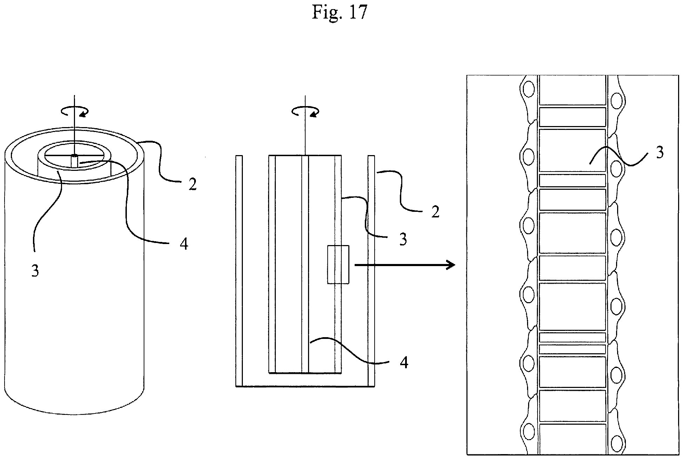

Figure 17 shows a possibility of moving the outer surface of the tubular membrane 3 relative to the inner surface of the tubular outer electrode 2. As can be seen in the picture on the left and in the middle the tubular membrane 3 can rotate or oscillate around its longitudinal axis. If cells are cultured on one or both sides of the tubular membrane 3 (see the picture on the right, which is an enlarged detail of the picture in the middle), the cells are moved in relation to the quasi-stationary liquid filling the void between the tubular outer electrode 2 and the tubular membrane 3 and between the tubular membrane 3 and the inner electrode 4. As a consequence shear forces are exerted on the cells cultured on the surface of the tubular membrane.

### REFERENCE NUMBERS

- 1: arrangement
- 2: tubular outer electrode
- 3: capillary membrane (hollow fiber membranes)
- 4: inner electrode
- 5a,5b: fittings
- 6a: inlet port
- 6b: outlet port
- 7a,7b: protective sheathing
- 8: contacts
- 9: proximal end
- 10: distal end
- 11: fixation means

## Claims

1. Use of an arrangement (1) for impedance measurements across the surface of a tubular membrane manufactured from a semi-permeable material, the arrangement (1) comprising:
a) an tubular outer electrode (2) having a longitudinal axis;
b) at least one tubular membrane (3) manufactured from a semi-permeable membrane and having a longitudinal axis, the at least one tubular membrane (3) being located within the tubular outer electrode (2) in such a way that the longitudinal axis of the tubular membrane (3) is substantially parallel to the longitudinal axis of the tubular outer electrode (2);
c) at least one inner electrode (4) in the form of a wire, tube or rod having a longitudinal axis, the at least one inner electrode (4) being located within the at least one tubular membrane (3) in such a way that the longitudinal axis of the at least one inner electrode (4) is substantially parallel to the longitudinal axis of the at least one tubular membrane (3)
for analyzing the formation of deposits or biofilms on the surface sides of the semi-permeable membrane (3) or in a cell culture system.

2. Use of the arrangement (1) according to claim 1, wherein the tubular outer electrode (2), the at least one tubular membrane (3) and the at least one inner electrode (4) are dimensioned and arranged in such a way that the at least one tubular membrane (3) is sandwiched between the tubular outer electrode (2) and the at least one inner electrode (4) over at least 10 %, preferably at least 25 %, more preferably at least 50 %, more preferably at least 75 and most preferably at least 95 % of the total length of the at least one tubular membrane (3).

3. Use of the arrangement (1) according to claim 1 or 2, wherein
- the tubular outer electrode (2), the at least one tubular membrane (3) and the at least one inner electrode (4) have a length in the range from 0.5 cm to 200 cm, preferably from 2 cm to 20 cm,
- the tubular outer electrode (2) has an inner diameter in the range from 1 mm to 100 mm, preferably in the range from 3 mm to 20 mm,
- the at least one tubular membrane (3) has an inner diameter in the range from 10 µm to 49 mm, preferably in the range from 0.5 mm to 10 mm, and
- the at least one inner electrode (4) has an outer diameter in the range from 0.1 mm to 45 mm, preferably in the range from 0.2 mm to 2 mm.

4. Use of the arrangement (1) according to anyone of claims 1 to 3, wherein the tubular outer electrode (2) and the at least one inner electrode (4) are manufactured from or coated with an electrically conductive material selected from the group consisting of a metal, an alloy and an electrically conductive polymer.

5. Use of the arrangement (1) according to anyone of claims 1 to 4, wherein
- the semi-permeable membrane from which the at least one tubular membrane (3) is manufactured has a wall thickness in the range from 10 µm to 5 mm, preferably from 100 µm to 1 mm, and
- the semi-permeable membrane from which the at least one tubular membrane (3) is a porous semi-permeable membrane having a pore size in the range from 1 nm to 10 µm, preferably from 0.2 µm to 3 µm.

6. Use of the arrangement (1) according to anyone of claims 1 to 5, wherein the semi-permeable membrane from which the at least one tubular membrane (3) is manufactured is based on a material from the group consisting of a thermoplastic polymer, preferably polypropylene, polyethylene or polysulfone, cellulose or a cellulose derivative, preferably cellulose acetate and regenerated cellulose, sulfonated polyethersulfone polymers and fluorinated polymers, preferably polyvinylidene fluoride (PVDF) or polytetrafluoroethylene (PTFE, Teflon).

7. Use of the arrangement (1) according to anyone of claims 1 to 6, wherein the arrangement (1) further comprises fittings (5a, 5b) at the proximal end and the distal end of the arrangement (1), the fittings (5a, 5b) being connectable to a tube and forming the closure of the proximal and distal end of the tubular outer electrode (2).

8. Use of the arrangement (1) according to anyone of claims 1 to 7, wherein the arrangement (1) further comprises an inlet port (6a) located near the proximal end of the arrangement (1) and an outlet port (6b) being located near the distal end of the arrangement (1), inlet port (6a) and outlet port (6b) being configured to allow the entry (inlet port 6a) and the discharge (outlet 6b) of a liquid medium through the casing of the tubular outer electrode (2).

9. Use of the arrangement (1) according to anyone of claims 1 to 8, wherein the arrangement (1) further comprises at least one protective sheathing (7a) in the form of tubular membrane manufactured from a semi-permeable membrane, the at least protective sheathing (7a) covering the surface of the at least one inner electrode (4) and having a longitudinal axis, the at least one inner electrode (4) being located within the at least one protective sheathing (7a) and the at least one protective sheathing (7a) being located within the tubular membrane (3) and the axis of the at least one inner electrode (4), the tubular membrane (3) and the at least one protective sheathing (7a) being substantially parallel to each other.

10. Use of the arrangement (1) according to anyone of claims 1 to 9, wherein the arrangement (1) further comprises a protective sheathing (7b) in the form of tubular membrane manufactured from a semi-permeable membrane, the protective sheathing (7b) covering the inner surface of the tubular outer electrode (2) and having a longitudinal axis, the tubular membrane (3) being located within the protective sheathing (7b) and the protective sheathing (7b) being located within the tubular outer electrode (2) and the axis of the tubular outer electrode (2), the tubular membrane (3) and the protective sheathing (7b) being substantially parallel to each other.

11. Use of the arrangement (1) according to anyone of claims 1 to 10, wherein the arrangement (1) comprises a plurality of tubular capillaries (3) being located within the outer electrode (2), wherein in at least one of the tubular capillaries an inner electrode (4) is located.

12. A process for measuring the impedance across the surface of a tubular membrane (3) manufactured from a semi-permeable material, the process comprising the process step of:
A) providing an arrangement (1) as defined within any one of claims 1 to 11;
B) filling the void within the tubular outer electrode (2), the void within the at least one tubular membrane (3) and the pores of the semi-permeable material with an electrically conductive liquid;
C) measuring the impedance across the surface of the at least one tubular membrane (3);
wherein deposits or biofilms are formed on the surface sides of the semi-permeable membrane (3) or wherein cells are cultured on the surface side of the at least one tubular membrane (3) that is directed to the inner surface of the tubular outer electrode (2) and/or on the surface side that is directed to the at least one inner electrode (4).

13. The process according to claim 12, wherein the electrically conductive liquid flows through the outer electrode (2) and/or the at least one tubular membrane (3) while the impedance is measured.

14. The process according to claim 13, wherein the electrically conductive liquid flows through the outer electrode (2) and the at least one tubular membrane (3) in a counter flow direction or in a concurrent flow direction while the impedance is measured.

## Patentansprüche

1. Verwendung einer Anordnung (1) für Impedanzmessungen über die Oberfläche einer aus einem semipermeablen Werkstoff hergestellten röhrenförmigen Membran, wobei die Anordnung (1) Folgendes umfasst:
a) eine röhrenförmige Außenelektrode (2) mit einer Längsachse;
b) mindestens eine röhrenförmige Membran (3), die aus einer semipermeablen Membran hergestellt ist und eine Längsachse aufweist, wobei die mindestens eine röhrenförmige Membran (3) innerhalb der röhrenförmigen Außenelektrode (2) derart angeordnet ist, dass die Längsachse der röhrenförmigen Membran (3) im Wesentlichen parallel zur Längsachse der röhrenförmigen Außenelektrode (2) ist;
c) mindestens eine Innenelektrode (4) in Form eines Drahtes, einer Röhre oder eines Stabs mit einer Längsachse, wobei die mindestens eine Innenelektrode (4) innerhalb der mindestens einen röhrenförmigen Membran (3) derart angeordnet ist, dass die Längsachse der mindestens einen Innenelektrode (4) im Wesentlichen parallel zur Längsachse der mindestens einen röhrenförmigen Membran (3) ist,
zum Analysieren der Bildung von Ablagerungen oder Biofilmen auf den Oberflächenseiten der semipermeablen Membran (3) oder in einem Zellkultursystem.

2. Verwendung der Anordnung (1) nach Anspruch 1, wobei die röhrenförmige Außenelektrode (2), die mindestens eine röhrenförmige Membran (3) und die mindestens eine Innenelektrode (4) so dimensioniert und angeordnet sind, dass die mindestens eine röhrenförmige Membran (3) zwischen die röhrenförmige Außenelektrode (2) und die mindestens eine Innenelektrode (4) über mindestens 10%, vorzugsweise mindestens 25%, stärker bevorzugt mindestens 50%, stärker bevorzugt mindestens 75% und am stärksten bevorzugt mindestens 95% der Gesamtlänge der mindestens einen röhrenförmigen Membran (3) sandwichartig eingebracht ist.

3. Verwendung der Anordnung (1) nach Anspruch 1 oder 2, wobei
- die röhrenförmige Außenelektrode (2), die mindestens eine röhrenförmige Membran (3) und die mindestens eine Innenelektrode (4) eine Länge im Bereich von 0,5 cm bis 200 cm, vorzugsweise von 2 cm bis 20 cm, haben,
- die röhrenförmige Außenelektrode (2) einen Innendurchmesser im Bereich von 1 mm bis 100 mm, vorzugsweise im Bereich von 3 mm bis 20 mm, hat,
- die mindestens eine röhrenförmige Membran (3) einen Innendurchmesser im Bereich von 10 µm bis 49 mm, vorzugsweise im Bereich von 0,5 mm bis 10 mm, hat und
- die mindestens eine Innenelektrode (4) einen Außendurchmesser im Bereich von 0,1 mm bis 45 mm, vorzugsweise im Bereich von 0,2 mm bis 2 mm, hat.

4. Verwendung der Anordnung (1) nach einem der Ansprüche 1 bis 3, wobei die röhrenförmige Außenelektrode (2) und die mindestens eine Innenelektrode (4) aus einem elektrisch leitfähigen Werkstoff hergestellt oder damit beschichtet sind, der aus der aus einem Metall, einer Legierung und einem elektrisch leitfähigen Polymer bestehenden Gruppe ausgewählt ist.

5. Verwendung der Anordnung (1) nach einem der Ansprüche 1 bis 4, wobei
- die semipermeable Membran, aus der die mindestens eine röhrenförmige Membran (3) hergestellt ist, eine Wanddicke im Bereich von 10 µm bis 5 mm, vorzugsweise von 100 µm bis 1 mm, hat und
- die semipermeable Membran, aus der die mindestens eine röhrenförmige Membran (3), eine poröse semipermeable Membran mit einer Porengröße im Bereich von 1 nm bis 10 µm, vorzugsweise von 0,2 µm bis 3 µm, ist.

6. Verwendung der Anordnung (1) nach einem der Ansprüche 1 bis 5, wobei die semipermeable Membran, aus der die mindestens eine röhrenförmige Membran (3) herstellt ist, auf einem Werkstoff aus der aus einem thermoplastischen Polymer, vorzugsweise Polypropylen, Polyethylen oder Polysulfon, Cellulose oder einem Cellulosederivat, vorzugsweise Celluloseacetat und regenerierte Cellulose, sulfonierten Polyethersulfon-Polymeren und fluorierten Polymeren, vorzugsweise Polyvinylidenfluorid (PVDF) oder Polytetrafluorethylen (PTFE, Teflon), bestehenden Gruppe basiert.

7. Verwendung der Anordnung (1) nach einem der Ansprüche 1 bis 6, wobei die Anordnung (1) weiterhin Verbindungsstücke (5a, 5b) am proximalen Ende und am distalen Ende der Anordnung (1) aufweist, wobei die Verbindungsstücke (5a, 5b) mit einem Rohr verbindbar sind und den Verschluss des proximalen und distalen Endes der röhrenförmigen Außenelektrode (2) bilden.

8. Verwendung der Anordnung (1) nach einem der Ansprüche 1 bis 7, wobei die Anordnung (1) weiterhin eine in der Nähe des proximalen Endes der Anordnung (1) befindliche Einlassöffnung (6a) und eine in der Nähe des distalen Endes der Anordnung (1) befindliche Auslassöffnung (6b) umfasst, wobei Einlassöffnung (6a) und Auslassöffnung (6b) so konfiguriert sind, dass sie den Einstrom (Einlassöffnung 6a) und das Abfließen (Auslass 6b) eines flüssigen Mediums durch die Hülle der röhrenförmigen Außenelektrode (2) ermöglichen.

9. Verwendung der Anordnung (1) nach einem der Ansprüche 1 bis 8, wobei die Anordnung (1) weiterhin mindestens eine Schutzummantelung (7a) in Form einer aus einer semipermeablen Membran hergestellten röhrenförmigen Membran umfasst, wobei die mindestens eine Schutzummantelung (7a) die Oberfläche der mindestens einen Innenelektrode (4) bedeckt und eine Längsachse aufweist, wobei die mindestens eine Innenelektrode (4) innerhalb der mindestens einen Schutzummantelung (7a) angeordnet ist und die mindestens eine Schutzummantelung (7a) innerhalb der röhrenförmigen Membran (3) angeordnet ist und die Achse der mindestens einen Innenelektrode (4), der röhrenförmigen Membran (3) und der mindestens einen Schutzummantelung (7a) im Wesentlichen parallel zueinander sind.

10. Verwendung der Anordnung (1) nach einem der Ansprüche 1 bis 9, wobei die Anordnung (1) weiterhin eine Schutzummantelung (7b) in Form einer aus einer semipermeablen Membran hergestellten röhrenförmigen Membran umfasst, wobei die Schutzummantelung (7b) die innere Oberfläche der röhrenförmigen Außenelektrode (2) bedeckt und eine Längsachse aufweist, wobei die röhrenförmige Membran (3) innerhalb der Schutzummantelung (7b) angeordnet ist und die Schutzummantelung (7b) innerhalb der röhrenförmigen Außenelektrode (2) angeordnet ist und die Achse der röhrenförmigen Außenelektrode (2), der röhrenförmigen Membran (3) und der Schutzummantelung (7b) im Wesentlichen parallel zueinander sind.

11. Verwendung der Anordnung (1) nach einem der Ansprüche 1 bis 10, wobei die Anordnung (1) eine Mehrzahl an röhrenförmigen Kapillaren (3) aufweist, die innerhalb der Außenelektrode (2) angeordnet sind, wobei sich in mindestens einer der röhrenförmigen Kapillaren eine Innenelektrode (4) befindet.

12. Verfahren zum Messen der Impedanz über die Oberfläche einer aus einem semipermeablen Werkstoff hergestellten röhrenförmigen Membran (3), wobei das Verfahren den folgenden Verfahrensschritt umfasst:
A) Bereitstellen einer Anordnung (1) nach einem der Ansprüche 1 bis 11;
B) Füllen des Hohlraums innerhalb der röhrenförmigen Außenelektrode (2), des Hohlraums innerhalb der mindestens einen röhrenförmigen Membran (3) und die Poren des semipermeablen Werkstoffs mit einer elektrisch leitfähigen Flüssigkeit;
C) Messen der Impedanz über die Oberfläche der mindestens einen röhrenförmigen Membran (3),
wobei sich Ablagerungen oder Biofilme auf den Oberflächenseiten der semipermeablen Membran (3) bilden oder wobei Zellen auf der Oberflächenseite der mindestens einen röhrenförmigen Membran (3), die zur inneren Oberfläche der röhrenförmigen Außenelektrode (2) hin zeigt, und/oder auf der Oberflächenseite, die zu der mindestens einen Innenelektrode (4) hin zeigt, gezüchtet werden.

13. Verfahren nach Anspruch 12, wobei die elektrisch leitfähige Flüssigkeit durch die Außenelektrode (2) und/oder die mindestens eine röhrenförmige Membran (3) fließt, während die Impedanz gemessen wird.

14. Verfahren nach Anspruch 13, wobei die elektrisch leitfähige Flüssigkeit durch die Außenelektrode (2) und die mindestens eine röhrenförmige Membran (3) in einer Gegenströmungsrichtung oder in einer Gleichströmungsrichtung fließt, während die Impedanz gemessen wird.

## Revendications

1. Utilisation d'un agencement (1) pour des mesures d'impédance à travers la surface d'une membrane tubulaire fabriquée à partir d'un matériau semi-perméable, l'agencement (1) comprenant :
a) une électrode extérieure tubulaire (2) ayant un axe longitudinal ;
b) au moins une membrane tubulaire (3) fabriquée à partir d'une membrane semi-perméable et ayant un axe longitudinal, l'au moins une membrane tubulaire (3) étant située à l'intérieur de l'électrode extérieure tubulaire (2) de telle sorte que l'axe longitudinal de la membrane tubulaire (3) est substantiellement parallèle à l'axe longitudinal de l'électrode extérieure tubulaire (2) ;
c) au moins une électrode intérieure (4) sous la forme d'un fil, d'un tube ou d'une tige ayant un axe longitudinal, l'au moins une électrode intérieure (4) étant située à l'intérieur de l'au moins une membrane tubulaire (3) de telle sorte que l'axe longitudinal de l'au moins une électrode intérieure (4) est substantiellement parallèle à l'axe longitudinal de l'au moins une membrane tubulaire (3)
pour analyser la formation de dépôts ou de biofilms sur les côtés de la surface de la membrane semi-perméable (3) ou dans un système de culture cellulaire.

2. Utilisation de l'agencement (1) selon la revendication 1, dans laquelle l'électrode extérieure tubulaire (2), l'au moins une membrane tubulaire (3) et l'au moins une électrode intérieure (4) sont dimensionnées et agencées de telle sorte que l'au moins une membrane tubulaire (3) est intercalée entre l'électrode extérieure tubulaire (2) et l'au moins une électrode intérieure (4) sur au moins 10 %, de préférence au moins 25 %, mieux au moins 50 %, mieux encore au moins 75 % et idéalement au moins 95 % de la longueur totale de l'au moins une membrane tubulaire (3).

3. Utilisation de l'agencement (1) selon la revendication 1 ou 2, dans laquelle
- l'électrode extérieure tubulaire (2), l'au moins une membrane tubulaire (3) et l'au moins une électrode intérieure (4) ont une longueur dans la gamme de 0,5 cm à 200 cm, de préférence de 2 cm à 20 cm,
- l'électrode extérieure tubulaire (2) a un diamètre intérieur dans la gamme de 1 mm à 100 mm, de préférence dans la gamme de 3 mm à 20 mm,
- l'au moins une membrane tubulaire (3) a un diamètre intérieur dans la gamme de 10 µm à 49 mm, de préférence dans la gamme de 0,5 mm à 10 mm, et
- l'au moins une électrode intérieure (4) a un diamètre extérieur dans la gamme de 0,1 mm à 45 mm, de préférence dans la gamme de 0,2 mm à 2 mm

4. Utilisation de l'agencement (1) selon l'une quelconque des revendications 1 à 3, dans laquelle l'électrode extérieure tubulaire (2) et l'au moins une électrode intérieure (4) sont fabriquées à partir de ou recouvertes avec un matériau électriquement conducteur choisi dans le groupe constitué par un métal, un alliage et un polymère électriquement conducteur.

5. Utilisation de l'agencement (1) selon l'une quelconque des revendications 1 à 4, dans laquelle
- la membrane semi-perméable à partir de laquelle l'au moins une membrane tubulaire (3) est fabriquée a une épaisseur de paroi dans la gamme de 10 µm à 5 mm, de préférence de 100 µm à 1 mm, et
- la membrane semi-perméable à partir de laquelle l'au moins une membrane tubulaire (3) est une membrane semi-perméable poreuse ayant une taille de pores dans la gamme de 1 nm à 10 µm, de préférence de 0,2 µm à 3 µm.

6. Utilisation de l'agencement (1) selon l'une quelconque des revendications 1 à 5, dans laquelle la membrane semi-perméable à partir de laquelle l'au moins une membrane tubulaire (3) est fabriquée est basée sur un matériau issu du groupe constitué par un polymère thermoplastique, de préférence le polypropylène, le polyéthylène ou la polysulfone, la cellulose ou un dérivé de cellulose, de préférence l'acétate de cellulose et la cellulose régénérée, les polymères de polyéthersulfone sulfonés et les polymères fluorés, de préférence le polyfluorure de vinylidène (PVDF) ou le polytétrafluoroéthylène (PTFE, Téflon).

7. Utilisation de l'agencement (1) selon l'une quelconque des revendications 1 à 6, l'agencement (1) comprenant en outre des raccords (5a, 5b) à l'extrémité proximale et l'extrémité distale de l'agencement (1), les raccords (5a, 5b) étant raccordables à un tube et constituant la fermeture de l'extrémité proximale et distale de l'électrode extérieure tubulaire (2).

8. Utilisation de l'agencement (1) selon l'une quelconque des revendications 1 à 7, l'agencement (1) comprenant en outre un orifice d'entrée (6a) situé près de l'extrémité proximale de l'agencement (1) et un orifice de sortie (6b) situé près de l'extrémité distale de l'agencement (1), l'orifice d'entrée (6a) et l'orifice de sortie (6b) étant configurés pour permettre l'entrée (orifice d'entrée 6a) et l'évacuation (sortie 6b) d'un milieu liquide à travers l'enveloppe de l'électrode extérieure tubulaire (2).

9. Utilisation de l'agencement (1) selon l'une quelconque des revendications 1 à 8, l'agencement (1) comprenant en outre au moins une gaine protectrice (7a) sous la forme d'une membrane tubulaire fabriquée à partir d'une membrane semi-perméable, l'au moins une gaine protectrice (7a) recouvrant la surface de l'au moins une électrode intérieure (4) et ayant un axe longitudinal, l'au moins une électrode intérieure (4) étant située à l'intérieur de l'au moins une gaine protectrice (7a) et l'au moins une gaine protectrice (7a) étant située à l'intérieur de la membrane tubulaire (3) et les axes de l'au moins une électrode intérieure (4), de la membrane tubulaire (3) et de l'au moins une gaine protectrice (7a) étant substantiellement parallèles les uns aux autres.

10. Utilisation de l'agencement (1) selon l'une quelconque des revendications 1 à 9, l'agencement (1) comprenant en outre une gaine protectrice (7b) sous la forme d'une membrane tubulaire fabriquée à partir d'une membrane semi-perméable, la gaine protectrice (7b) recouvrant la surface intérieure de l'électrode extérieure tubulaire (2) et ayant un axe longitudinal, la membrane tubulaire (3) étant située à l'intérieur de la gaine protectrice (7b) et la gaine protectrice (7b) étant située à l'intérieur de l'électrode extérieure tubulaire (2) et les axes de l'électrode extérieure tubulaire (2), de la membrane tubulaire (3) et de la gaine protectrice (7b) étant sensiblement parallèles les uns aux autres.

11. Utilisation de l'agencement (1) selon l'une quelconque des revendications 1 à 10, l'agencement (1) comprenant une pluralité de capillaires tubulaires (3) situés à l'intérieur de l'électrode extérieure (2), une électrode intérieure (4) étant située dans au moins un des capillaires tubulaires.

12. Procédé de mesure de l'impédance à travers la surface d'une membrane tubulaire (3) fabriquée à partir d'un matériau semi-perméable, le procédé comprenant les étapes de procédé consistant à :
A) se procurer un agencement (1) tel que défini dans l'une quelconque des revendications 1 à 11 ;
B) remplir le vide à l'intérieur de l'électrode extérieure tubulaire (2), le vide à l'intérieur de l'au moins une membrane tubulaire (3) et les pores du matériau semi-perméable avec un liquide électriquement conducteur ;
C) mesurer l'impédance à travers la surface de l'au moins une membrane tubulaire (3) ;
dans lequel des dépôts ou des biofilms sont formés sur les côtés de la surface de la membrane semi-perméable (3) ou dans lequel des cellules sont cultivées sur le côté de la surface de l'au moins une membrane tubulaire (3) qui est dirigé vers la surface intérieure de l'électrode extérieure tubulaire (2) et/ou sur le côté de la surface qui est dirigée vers l'au moins une électrode intérieure (4).

13. Procédé selon la revendication 12, dans lequel le liquide électriquement conducteur circule à travers l'électrode extérieure (2) et/ou l'au moins une membrane tubulaire (3) pendant que l'impédance est mesurée.

14. Procédé selon la revendication 13, dans lequel le liquide électriquement conducteur circule à travers l'électrode extérieure (2) et l'au moins une membrane tubulaire (3) dans des directions à contre-courant ou des directions à co-courant pendant que l'impédance est mesurée.
